# EUROPÄISCHE PATENTSCHRIFT

(11) **EP 3 260 865 B1**
(45) Veröffentlichungstag und Bekanntmachung des Hinweises auf die Patenterteilung: **28.12.2022**
(21) Anmeldenummer: 17172222.6
(22) Anmeldetag: 05.04.2013
(51) Int. Cl.: G01N 33/68, C07K 7/04

(54) **VERFAHREN ZUR BEHANDLUNG VON BLUT, BLUTPRODUKTEN UND ORGANEN**
METHOD FOR THE TREATMENT OF BLOOD, BLOOD PRODUCTS AND ORGANS
PROCÉDÉ DE TRAITEMENT DU SANG, PRODUITS SANGUINS ET ORGANES

(30) Priorität: 05.04.2012 DE 102012102999; 14.09.2012 DE 102012108598; 14.09.2012 DE 102012108599
(43) Veröffentlichungstag der Anmeldung: 27.12.2017
(62) Teilanmeldung aus: 13718522.9
(73) Patentinhaber: Priavoid GmbH, 40225 Düsseldorf (DE)
(72) Erfinder: Willbold, Dieter, 52425 Jülich (DE)
(74) Vertreter: Fitzner, Uwe

(56) Entgegenhaltungen:
- WO-A2-02/42462
- WO-A2-02/081505
- WO-A2-2007/047967
- WO-A2-2010/062570
- WO-A2-2011/147797
- RANJINI K. SUNDARAM ET AL: "Novel Detox Gel Depot Sequesters &bgr;-Amyloid Peptides in a Mouse Model of Alzheimer's Disease", INTERNATIONAL JOURNAL OF PEPTIDE RESEARCH AND THERAPEUTICS, Bd. 18, Nr. 2, 23. November 2011 (2011-11-23), Seiten 99-106, XP055070073, ISSN: 1573-3149, DOI: 10.1007/s10989-011-9283-7
- FUNKE SUSANNE AILEEN ET AL: "Peptides for Therapy and Diagnosis of Alzheimer's Disease", CURRENT PHARMACEUTICAL DESIGN, BENTHAM SCIENCE PUBLISHERS, NL, Bd. 18, Nr. 6, 1. Februar 2012 (2012-02-01), Seiten 755-767, XP009170739, ISSN: 1381-6128
- ANDREAS MÜLLER-SCHIFFMANN ET AL: "Combining Independent Drug Classes into Superior, Synergistically Acting Hybrid Molecules", ANGEWANDTE CHEMIE INTERNATIONAL EDITION, Bd. 49, Nr. 46, 8. November 2010 (2010-11-08), Seiten 8743-8746, XP055083717, ISSN: 1433-7851, DOI: 10.1002/anie.201004437
- ZHANG G ET AL: "Multiple-Peptide Conjugates for Binding ß-Amyloid Plaques of Alzheimer's Disease", BIOCONJUGATE CHEMISTRY,, Bd. 14, 1. Januar 2003 (2003-01-01), Seiten 86-92, XP002458160, ISSN: 1043-1802, DOI: 10.1021/BC025526I
- SUSANNE AILEEN FUNKE ET AL: "Mirror image phage display-a method to generate d-peptide ligands for use in diagnostic or therapeutical applications", MOLECULAR BIOSYSTEMS, Bd. 5, Nr. 8, 1. Januar 2009 (2009-01-01), Seite 783, XP055069250, ISSN: 1742-206X, DOI: 10.1039/b904138a
- THOMAS VAN GROEN ET AL: "Reduction of Alzheimer's Disease Amyloid Plaque Load in Transgenic Mice by D3, a D-Enantiomeric Peptide Identified by Mirror Image Phage Display", CHEMMEDCHEM, Bd. 3, Nr. 12, 15. Dezember 2008 (2008-12-15), Seiten 1848-1852, XP055239322, ISSN: 1860-7179, DOI: 10.1002/cmdc.200800273
- SUSANNE AILEEN FUNKE ET AL: "-Enantiomeric Peptide D3 Improves the Pathology and Behavior of Alzheimer's Disease Transgenic Mice", ACS CHEMICAL NEUROSCIENCE, Bd. 1, Nr. 9, 15. September 2010 (2010-09-15), Seiten 639-648, XP055239332, US ISSN: 1948-7193, DOI: 10.1021/cn100057j

## Beschreibung

Die vorliegende Erfindung betrifft ein Verfahren zur Behandlung (Ex vivo) von Blut, Blutprodukten und Organen außerhalb des menschlichen oder tierischen Körpers indem Amyloid-Beta-Oligomere entfernt und/oder enttoxifiziert werden..

Aufgrund der demographischen Entwicklung in den nächsten Jahrzehnten wird sich die Zahl der Personen, die an altersbedingten Erkrankungen leiden, erhöhen. Hier ist insbesondere die sog. Alzheimer-Krankheit (AD, Alzheimersche Demenz, lateinisch = Morbus Alzheimer) zu nennen.

Ein Merkmal der Alzheimer-Erkrankung sind extrazelluläre Ablagerungen des Amyloid-Beta-Peptids (A-Beta-Peptid, Aß, oder Aß-Peptid). Diese Ablagerung des A-Beta-Peptids in Plaques ist typischerweise in den Gehirnen von AD-Patienten *post mortem* festzustellen. Deshalb werden verschiedene Formen des A-Beta-Peptids - wie z.B. Fibrillen - für die Entstehung und das Fortschreiten der Krankheiten verantwortlich gemacht. Zusätzlich werden seit einigen Jahren die kleinen, frei diffundierbaren A-Beta-Oligomere als hauptsächliche Verursacher der Entstehung und des Fortschritts der AD gesehen.

A-Beta-Monomere, als Bausteine der A-Beta-Oligomeren entstehen im menschlichen Körper ständig und sind vermutlich *per se* nicht toxisch. A-Beta-Monomere können sich in Abhängigkeit von ihrer Konzentration zufällig zusammen lagern. Die Konzentration ist abhängig von ihrer Bildungs- und Abbaurate im Körper. Findet mit zunehmendem Alter eine Erhöhung der Konzentration an A-Beta-Monomeren im Körper statt, ist eine spontane Zusammenlagerung der Monomere zu A-Beta-Oligomeren immer wahrscheinlicher. Die so entstandenen A-Beta-Oligomeren könnten sich analog zu den Prionen vermehren und letztendlich zur Morbus Alzheimer-Krankheit führen.

Ein wichtiger Unterschied zwischen Prävention und Behandlung oder gar Heilung der AD liegt in der Tatsache, dass eine Prävention schon durch die Verhinderung der Bildung der ersten A-Beta-Oligomere erreicht werden kann. Hierzu sind einige, wenige A-Beta-Liganden ausreichend, die wenig affin und selektiv bezüglich der A-Beta-Oligomere sind.

Die Bildung der A-Beta-Oligomere aus vielen Monomeren ist eine Reaktion hoher Ordnung und damit in hoher Potenz von der A-Beta-Monomer-Konzentration abhängig. Somit führt schon eine kleine Verringerung der aktiven A-Beta-Monomer-Konzentration zu einer Verhinderung der Bildung der ersten A-Beta-Oligomeren. Auf diesem Mechanismus basiert die bisherige Prävention. Bei der Behandlung von AD ist jedoch von einer völlig veränderten Situation auszugehen. Hier liegen nämlich A-Beta-Oligomere oder evtl. auch schon größere Polymere oder Fibrillen vor, die durch die Prionen-ähnliche Vermehrung der Oligomeren entstanden sind. Dies ist jedoch eine Reaktion niederer Ordnung und kaum noch von der A-Beta-Monomer-Konzentration abhängig.

Bisher existiert kein zugelassenes Medikament für eine ursächliche Behandlung der Alzheimerschen Demenz (AD). Typischerweise findet man in den Gehirnen von AD-Patienten *post-mortem* Ablagerungen des sogenannten beta-Amyloid-Peptides (Aß oder A-Beta) in Plaques. Deshalb werden schon lange verschiedene Formen des Aß-Oligomeren, z.B: Fibrillen, für die Entstehung und das Fortschreiten von AD verantwortlich gemacht. Seit wenigen Jahren werden besonders die kleinen, frei diffundierbaren Aß-Oligomere als hauptsächliche Verursacher für die Entstehung und den Fortschritt der AD verantwortlich gemacht. Aß-Monomere entstehen ständig in dem menschlichen Körper und sind vermutlich für sich gesehen nicht toxisch. Es wird spekuliert, ob sich Aß-Monomere abhängig von ihrer Konzentration zufällig und damit mit zunehmendem Alter immer wahrscheinlicher spontan zu Aß-Oligomeren zusammen lagern. Einmal entstandene Aß-Oligomere könnten sich durch eine Prion-ähnlichen Mechanismus vermehren und letzten Endes zur Krankheit führen. Seit einiger Zeit wird diskutiert, ob AD-ähnlich wie Prion-Krankheiten prinzipiell von Mensch zu Mensch übertragbar ist. Ähnliches gilt für alle Amyloid-assoziierten Krankheiten (z.B. Parkinson) zu. Insbesondere eine mögliche Übertragung durch Bluttransfusion, Verabreichung von Blutprodukten und Organtransplantationen könnte ohne geeignete Tests und Präventionsmethoden zu einer massiven Gefährdung der Gesundheit von Empfängern führen. In diesem Zusammenhang wurde in der nicht-wissenschaftlichen Literatur von der vorzeitigen Erkrankung an AD einer transgenen Maus berichtet, nachdem deren komplettes Blut gegen das einer erkrankten Maus ausgetauscht wurde.

Aufgrund dieser Überlegungen sollte es eine Möglichkeit geben, Blut, Blutprodukte und Organe durch (prophylaktische oder präventive) Behandlung von infektiösen Partikeln zu befreien, bzw. diese zu inaktivieren. Es sollte dabei das Ziel sein, toxische Aß-Oligomere vollständig zu entfernen oder zu vernichten, also zu enttoxifizieren und damit deren Prion-ähnliche Vermehrung zu verhindern.

Aus dem Stand der Technik sind verschiedene Methoden zur Eliminierung von bioschädlichen Stoffen, Biopartikeln, Molekülen und pathologischen Proteinablagerungen bekannt. So gibt es Forschungen, nach welchen Nanomagnete eingesetzt werden, um Blut in wenigen Minuten gezielt von einem Giftstoff zu reinigen. Ebenso ist beschrieben worden, durch direkte Absorption von Lipoproteinen (DALI) LDL-Cholesterin aus dem Blut zu entfernen.

Die Immobilisierung von Antikörpern oder Peptiden wird in der DE 600 26 983 T2 oder US 5,968,820 beschrieben.

Ferner ist aus der DE 102009037015 A1 eine Vorrichtung und ein Verfahren zur Eliminierung von bioschädlichen Stoffen aus Körperflüssigkeiten bekannt. Die Isolation von Zellen, Biopartikeln oder Molekülen aus Flüssigkeiten wird in der DE 102005063175 A1 beschrieben. Ferner ist aus der DE 102005031429 A1 ein Verfahren zur selektiven Bestimmung pathologischer Proteinablagerungen bekannt. Schließlich werden in der DE 102005009909 A1 Verbindungen zur Behandlung von Erkrankungen im Zusammenhang mit fehl gefalteten Proteinen beschrieben.

Die aus dem Stand der Technik bekannten Substanzen verringern die Konzentration von A-Beta-Monomeren und/oder Oligomeren auf verschiedenste Art und Weise. So sind z.B. Gamma-Sekretase-Modulatoren bekannt, die im Tierversuch zur Prävention eingesetzt wurden.

Aus der WO 02/081505 und DE 101 17 281 A1 sind verschiedene Sequenzen von D-Aminosäuren bekannt, die an A-Beta-Peptide binden. Diese Sequenzen der WO 02/081505 aus D-Aminosäuren binden mit einer Dissoziationskonstante K_{D}-Wert von 4 µM an Amyloid-Beta-Peptide.

Aus der WO 2011/147797 sind Hybridverbindungen bekannt, bestehend aus Aminopyrazolen und Peptiden, die A-Beta-Oligomerisation verhindern.

Eine Verwendung dieser Verbindungen zur Aufreinigung von Blut, Blutprodukten und/oder Organen wird jedoch nicht offenbart.

Bei vielen Substanzen, die im Tierversuch positive Ergebnisse gezeigt haben, konnte diese Wirkung in klinischen Studien am Menschen nicht bestätigt werden. In klinischen Studien der Phase II und III dürfen nur mit klar AD diagnostizierte Menschen behandelt werden. Hier reicht eine geringe Verringerung der A-Beta-Monomerkonzentration nicht mehr aus, um größere Mengen an A-Beta-Oligomeren zu verhindern, und/oder Einfluss auf den Krankheitsverlauf zu nehmen.

Bisher wird die Alzheimersche Demenz hauptsächlich durch neuro-psychologische Tests diagnostiziert, durch Experimente an Personen, bei denen die Symptome schon erkannt wurden. Es ist jedoch bekannt, dass A-Beta-Oligomere und die zeitlich darauf folgenden Fibrillen und Plaques bis zu 20 Jahre vor dem Auftreten der Symptome im Gehirn der Patienten entstehen, und schon irreversible Schäden verursacht haben können. Allerdings gibt es bisher noch keine Möglichkeit, die AD vor dem Ausbruch der Symptome zu diagnostizieren.

Ferner offenbart die WO 2010/062570 A2 eine Zusammensetzung zur Behandlung und / oder Vorbeugung von Alzheimer-Krankheit.

Sundaram et al. INTERNATIONAL JOURNAL OF PEPTIDE RESEARCH, 18(2) 2012 betrifft das Retro-inverse Peptid, ffvlk, das künstliche Fibrillen aus Aß mit mäßiger Affinität bindet.

Die WO 2011/147797 A2 betrifft eine Hybridverbindung auf der Basis von Aminopyrazolderivaten und Peptiden zur Verwendung als Therapeutikum bei der Behandlung von Krankheiten.

Funke et al. CURRENT PHARMACEUTICAL DESIGN, 18(6) 2012 beschreibt Peptide, die für die Diagnose und Therapie von AD entwickelt wurden, und die Vor- und Nachteile von Peptid-Medikamenten.

Die WO 02/081505 A2 offenbart Peptide, die an das Peptid Beta-Amyloid mit hoher Bindungsaffinität binden.

Müller-Schiffmann et al. ANGEW. CHEMIE INTERNATIONAL EDITION, 49(46), 2010 beschreibt Hybridverbindungen, bestehend aus einem organischen D-enantiomeren β-sheet-auflösenden Teil und einem, D-enantiomeren Aβ-erkennenden Peptidteil.

Zhang G. et al. Bioconjugate Chemistry, 14(1), 2003 offenbart, dass die Bildung von β-Amyloid-Plaques bei Alzheimer durch den intermolekularen Kontakt der 5-Aminosäuresequenz KLVFF in β-Amyloid-Peptiden mit einer Größe von 40 bis 43 Resten ausgelöst wird.

Aufgabe der vorliegenden Erfindung ist es nunmehr Blut, Blutprodukte und/oder Organe durch Behandlung von toxischen und/oder infektiösen Partikeln zu befreien bzw. diese zu inaktivieren. Es ist das Ziel die in Blut, Blutprodukten oder Organen vorhandenen Aß-Oligomere, vollständig zu entfernen oder in ungefährliche Formen umzuwandeln.

Gegenstand der Erfindung ist demgemäß Verfahren zur Behandlung (in vitro, ex vivo) von Blut, Blutprodukten und/oder Organen außerhalb des menschlichen oder tierischen Körpers indem Amyloid-Beta-Oligomere entfernt und/oder enttoxifiziert werden, dadurch gekennzeichnet, dass eine Verbindung enthaltend oder bestehend aus Peptid RD2, mit der Sequenz ptlhthnrrrrr gemäß SEQ ID NO: 66 eingesetzt wird.

Das behandelte Blut kann aus einer Blutbank stammen und/oder nach der Behandlung in einer Blutbank gelagert werden.

Ferner ist es möglich, das erfindungsgemäße Verfahren bei gesunden Personen präventiv anzuwenden. Dabei wird das Blut dieser Personen, die keine AD-Symptome aufweisen, von eventuell vorhandenen Amyloid-beta-Oligomeren befreit, ohne Bezug auf Verfahrensschritte zur therapeutischen Behandlung des menschlichen oder tierischen Körpers.

Die Aß-Oligomere können entfernt werden, indem z.B. dem Blut Beads hinzugefügt werden, die Aß-Oligomere binden oder zurück halten. Zur Behandlung von Blutproben können dafür die Aß-Oligomere bindenden Substanzen an magnetische Beads gebunden werden. Ein Beispiel für Beads sind Dynabeads der Firma Dynal. Dynabeads^{®} M-280 Streptavidin (Dynal A.S., Oslo) sind supramagnetische Mikrokugeln, die mit aufgereinigtem Streptavidin, welches Biotin mit hoher Affinität (K_{D} = 10⁻¹⁵) bindet, kovalent verknüpft sind. Nachfolgend können diese Beads mit den daran gebunden Aß-Oligomeren wieder entfernt werden, z.B. durch Affinitätschromatographie, Filtration, Größenausschluss Sedimentation oder Zentrifugation.

In einer Variante der Erfindung können die Aß-Oligomere entfernt werden, indem das Blut und/oder die Blutprobe über eine Oberfläche geleitet wird, die Aß-Oligomere bindet oder zurück hält. Erfindungsgemäß können die Moleküle, die Aß-Oligomer binden bzw. entoxifizieren (sogenannte Fängermoleküle) auf einem Träger angeordnet sein, über den die flüssige Probe geleitet wird. In einer Variante ist auch für die Fängermoleküle eine Immobilisierung mit Hilfe von Nanomagneten möglich. Ebenso ist die Anordnung der Fängermoleküle in einem Dialyse-System möglich. In einer weiteren Variante können die Fängermoleküle aus einem biokompatiblen Material bestehen. Träger können auch Membranen, Filter, Filterschwämme, Beads, Stäbe, Seile, Säulen und Hohlfasern sein.

In einer weiteren Variante betreffend Organe können die Aß-Oligomere entfernt werden, indem die Fängermölekule über und/oder durch ein Organ geleitet werden (in vitro / ex vivo).

In einer weiteren Variante der Erfindung können Aß-Oligomere inaktiviert werden, indem eine Substanz zugefügt wird, die Aß-Oligomere in nicht-toxische, nicht-amyloidogene, nicht-infektiöse Formen umwandelt.

Als Aß-Oligomere-inaktivierende Substanzen oder als Aß-Oligomere-bindende Substanzen wird eine Verbindung enthaltend oder bestehend aus Peptid RD2, mit der Sequenz ptlhthnrrrrr gemäß SEQ ID NO: 66 eingesetzt . Die einzusetzenden Substanzen sollen eine möglichst hohe Affinität zu Aß-Oligomeren besitzen. Die entsprechende Dissoziationskonstante soll im µM-Bereich, bevorzugt nM-Bereich, insbesondere pM-Bereich oder noch tiefer liegen. Da das Zielmolekül der therapeutischen Behandlung ein Aß-Oligomer und damit natürlicher Weise ein multivalentes Target ist, kann in einer Variante der Erfindung für die Behandlung die einzusetzende Substanz aus mehreren Kopien einer bereits effizient Aß-Oligomer-bindenden Einheit hergestellt werden. Bei Abwesenheit störende Einflüsse (z.B. durch sterische Behinderung) kann ein n-mer einer Aß-Oligomer-bindenden Einheit, die an Aß-Oligomer mit einer Dissoziationskonstante (K_{D}) von mindestens x bindet, eine apparente K von xⁿ erreicht werden. Um dies zu erreichen existieren verschiedene Möglichkeiten: die Aß-Oligomer-bindenden Einheiten (Monomereinheiten) können kovalent oder nicht-kovalent (z.B. eine Biotin-Gruppe oder einen Streptavdin-Tetramer) miteinander verknüpft werden. In einer Variante der Erfindung können beliebig viele Kopien auf der Oberfläche von Beads immobilisiert werden.

Im Sinne der vorliegenden Erfindung bezeichnet der Begriff A-Beta-Oligomere sowohl A-Beta-Aggregate als auch A-Beta-Oligomere und auch kleine frei diffundierende A-Beta-Oligomere. Oligomer im Sinne der Erfindung ist ein Polymer gebildet aus 2, 3, 4, 5, 6, 7, 8, 9, 10, 11, 12, 13, 14, 15, 16, 17, 18, 19 oder 20 Monomeren oder Vielfachen davon.

Das vorliegende Verfahren wird außerhalb des menschlichen oder tierischen Körpers durchgeführt. Die hierfür verwendeten Begriffe sind in vitro oder ex vivo.

Bei den Polymeren handelt es sich um Peptide. Diese bestehen aus D-Aminosäuren.

Im Sinne der vorliegenden Erfindung bedeutet der Begriff "im Wesentlichen aus D-Aminosäuren", dass die einzusetzenden Monomere mindestens 60%, bevorzugt 75%, 80%, besonders bevorzugt 85%, 90%, 95%, insbesondere 96%, 97%, 98%, 99%, 100% aus D-Aminosäuren aufgebaut sind.

In einer Variante werden Monomere enthaltend oder bestehend aus Peptid RD2, mit der Sequenz ptlhthnrrrrr gemäß SEQ ID NO: 66 eingesetzt, die an ein A-Beta-Monomer und/oder A-Beta-Oligomere und/oder Fibrillen des A-Beta-Peptids mit einer Dissoziationskonstante (K_{D}-Wert) von höchstens 500 µM, bevorzugt 250, 100, 50 µM, besonders bevorzugt 25, 10, 6 µM, insbesondere 4 µM binden.

Das Polymer kann 2, 3, 4, 5, 6, 7, 8, 9, 10 oder mehr der oben beschriebenen Monomere enthalten.

In einer weiteren nicht erfindungsgemäßen Ausführung werden die Monomere ausgewählt aus der Gruppe bestehend aus:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79 sowie Homologe davon.

In einer weiteren Variante binden die Polymere an die Multimerisierungsdomäne des Amyloid beta-Peptids.

Der Begriff "Multimerisierungsdomäne" definiert diejenigen Domänen des Amyloid beta-Peptids, die mit der Interaktion der Amyloid beta-Peptide untereinander zu tun haben. In einer Variante erfüllen die Aminosäuren 10-42 des Amyloid beta-Peptids diese Funktion.

In einer weiteren nicht erfindungsgemäßen Variante weisen die Monomere Sequenzen auf, die sich von den angegebenen Sequenzen um bis zu drei Aminosäuren unterscheiden.

Ferner können als nicht erfindungsgemäße Monomere auch Sequenzen eingesetzt werden, die die oben genannten Sequenzen enthalten.

In einer weiteren nicht erfindungsgemäßen Variante weisen die Monomere Fragmente der oben genannten Sequenzen auf oder weisen homologe Sequenzen zu den oben genannten Sequenzen auf.

"Homologe Sequenzen" oder "Homologe" bedeutet im Sinne der Erfindung, dass eine Aminosäuresequenz eine Identität mit einer der oben genannten Aminosäuresequenz der Monomere von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3. Zwei Aminosäuresequenzen sind im Sinne der vorliegenden Erfindung identisch wenn sie die selbe Aminosäuresequenz besitzen.

Unter Homologe sind in einer Variante die entsprechenden retro-inversen Sequenzen der oben genannten Monomere zu verstehen. Mit dem Begriff "retro-inverse Sequenz" wird erfindungsgemäß eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro = rückwärts).

Das Polymer ist aus identischen Monomeren aufgebaut, oder enthält verschiedene Monomere.

In einer nicht erfindungsgemäßen Alternative ist das Polymer aus jeder beliebige Kombination aus 2, 3, 4, 5, 6, 7, 8, 9, 10 oder mehr der oben beschriebenen Monomere aufgebaut.

In einer nicht erfindungsgemäßen Ausführung ist das Polymer ein Dimer aus zwei D3-Monomeren (SEQ ID NO:13).

In einer nicht erfindungsgemäßen Ausführung ist das Polymer ein Dimer aus zwei RD2-Monomeren (RD 2- RD 2: ptlhthnrrrrrptlhthnrrrrr (SEQ ID NO: 76).

Dimere können beispielsweise über chemische Synthese bzw. Peptidsynthese hergestellt werden.

In einer Ausführung der Erfindung sind die Monomere kovalent miteinander verknüpft. In einer weiteren nicht erfindungsgemäßen Ausführung sind die Monomere nicht kovalent miteinander verbunden.

Eine kovalente Verbindung bzw. Verknüpfung der Monomer-Einheiten liegt im Sinne der Erfindung vor, falls die Peptide Kopf an Schwanz linear miteinander verknüpft werden, ohne dass dazwischen Linker oder Linker-Gruppen eingesetzt werden.

Eine nicht kovalente Verknüpfung im Sinne der Erfindung liegt vor, falls die Monomere über Biotin und Streptavidin, insbesondere Streptavidin-Tetramer miteinander verknüpft sind.

Eine kovalente Verknüpfung kann erreicht werden, indem die Monomer-Einheiten Kopf an Kopf, Schwanz an Schwanz oder Kopf an Schwanz linear gekoppelt werden, jeweils ohne Linker oder mit einer Linker-Gruppe. In einer Alternative ist auch eine Verknüpfung in einem Baum-artigen Gerüst (Dendrimere) auf einem Plattform-Molekül oder einer Kombination dieser Optionen möglich. Hierbei können auch nicht-identische Monomer-Einheiten kombiniert werden.

Das Polymer ist dadurch gekennzeichnet, dass es Amyloid-Beta-Oligomere mit einer Dissoziationskonstante von höchstens 1 mM, bevorzugt 800, 600, 400, 200, 100, 10 µM, besonders bevorzugt 1000, 900, 800, 700, 600, 500, 400, 300, 200, 100, 50 nM, insbesondere bevorzugt, 1000, 900, 800, 700, 600, 500, 400, 300, 200, 100, 50 pM, am meisten bevorzugt höchstens 20 pM bindet.

Das Polymer ist geeignet zur Verwendung in der Medizin.

In einer Ausführung handelt es sich um ein Polymer, welches zur ex vivo Behandlung von Morbus Alzheimer eingesetzt werden kann. In einer weiteren Ausführung handelt es sich um ein Polymer, welches zur ex vivo Behandlung von Parkinson, Creutzfeldt Jakob Disease (CJD), Scrapie, bovine spongiforme Enzephalopatie (BSE), oder Diabetes eingesetzt werden kann.

Die aufgebauten Polymere aus Monomeren, die ihrerseits an A-Beta-Oligomeren binden, zeigen deutliche, synergetische Effekte in Bezug auf ihre Selektivität und Affinität zu den A-Beta-Oligomeren im Vergleich zu den Monomeren. Mit anderen Worten: Die erfindungsgemäßen Polymere sind den Monomeren überlegen. Synergetische Effekte im Sinne der vorliegenden Erfindung sind Effekte, die eine höhere Selektivität und Affinität bezüglich der A-Beta-Oligomeren zeigen, insbesondere des K_{D}-Wertes betreffend die Bindung an A-Beta-Oligomere als die Monomere einzeln oder in ihrer Addition.

Unter einem Linker sind ein oder mehrere Moleküle zu verstehen, die über kovalente Bindungen an die Monomere gebunden sind, wobei diese Linker untereinander auch durch kovalente Bindungen verknüpft sein können.

In einer Alternative werden durch die Linker die durch die Monomere vorgegebenen Eigenschaften des Polymers, nämlich die Bindung an A-Beta-Oligomere, nicht verändert.

In einer weiteren Alternative bewirken die Linker eine Veränderung der Eigenschaften des Polymers, die durch die Monomere vorgegeben sind. In einer solchen Ausführung wird die Selektivität und/oder Affinität der Polymere bezüglich der A-Beta-Oligomere verstärkt und/oder die Dissoziationskonstante herabgesetzt. In einer weiteren Ausführung werden die Linker so ausgewählt oder können so angeordnet sein, dass sie die sterische Wirkung der Polymere derart verändern, dass diese selektiv nur an A-Beta-Oligomere einer bestimmten Größe binden.

Eine solche Änderung der sterischen Wirkung der Polymere kann auch durch den Aufbau verzweigter Polymere, durch Dendrimere eines besonderen Aufbaus oder den entsprechenden Aufbau des Polymers mittels Monomere und einem Plattformmolekül oder Kombinationen dieser Optionen erreicht werden.

Weiterer, nicht erfindungsgemäßer Gegenstand der Offenbarung ist eine Zusammensetzung sowie deren Verwendung zur Bestimmung von Morbus Alzheimer, in der das D-Peptid
a) eine retro-inverse Sequenz des Amyloid beta-Peptids oder Amyloid beta-Peptid-Teilfragmenten enthält und vollständig aus D-Aminosäuren besteht und/oder
b) an die Multimerisierungsdomäne des Amyloid beta-Peptids bindet und/oder
c) die Sequenz SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79enthält oder aufweist und vollständig aus D-Aminosäuren besteht und/oder
d) D-Peptide mit der Sequenz SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID N0:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79enthält oder aufweist, wobei die D-Peptide teilweise L-Aminosäuren enthalten und/oder
e) homologe Sequenzen zu SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79enthält oder aufweist.

"D-Peptide" bestehen in einer Variante aus einer retro-inversen Sequenz zum Amyloid beta-Peptid oder Amyloid beta-Peptid-Teilfragmenten und vollständig aus D-Aminosäuren.

Ein "Teilfragment" besteht aus 3, 4, 5, 6, 7, 8, 9, 10, 11, 12, 13, 14, 15, 16 oder mehr Aminosäuren homolog zur Aminosäuresequenz des Amyloid beta-Peptids.

In einer weiteren nicht erfindungsgemäßen Variante binden die D-Peptide an die Multimerisierungsdomäne des Amyloid beta-Peptids. In einer weiteren Variante weisen die D-Peptide die Sequenz SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79 auf und bestehen vollständig aus D-Aminosäuren. In einer weiteren nicht erfindungsgemäßen Variante weisen die D-Peptide eine der oben genannten Sequenzen auf, und enthalten teilweise L-Aminosäuren. In einer weiteren nicht erfindungsgemäßen Variante weisen die D-Peptide homologe Sequenzen zu den oben genannten Sequenzen auf. Unter "D-Peptid" wird ein Peptid verstanden, welches sich aus Aminosäuren in der D-Form zusammensetzt.

In einer nicht erfindungsgemäßen Variante weisen die D-Peptide auch teilweise L-Aminosäuren auf. "Teilweise" L-Aminosäuren bedeutet, dass 1, 2, 3, 4, 5, 6, 7, 8, 9, 10 oder mehr Aminosäuren homolog zur Aminosäuresequenz des aus D-Aminosäuren bestehenden D-Peptids durch jeweils die gleiche Aminosäure in der L-Konformation ersetzt sind.

"D-Peptide" bestehen in einer nicht erfindungsgemäßen Variante aus einer retro-inversen Sequenz zum Amyloid beta-Peptid oder Amyloid beta-Peptid-Teilfragmenten und vollständig aus D-Aminosäuren.

Ein "Teilfragment" besteht aus 3, 4, 5, 6, 7, 8, 9, 10, 11, 12, 13, 14, 15, 16 oder mehr Aminosäuren homolog zur Aminosäuresequenz des Amyloid beta-Peptids.

In einer weiteren nicht erfindungsgemäßen Variante binden die erfindungsgemäßen D-Peptide an die Multimerisierungsdomäne des Amyloid beta-Peptids. In einer weiteren nicht erfindungsgemäßen Variante weisen die D-Peptide die oben genannten Sequenzen SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79auf und bestehen vollständig aus D-Aminosäuren.

In einer anderen nicht erfindungsgemäßen Ausführungsform umfasst das Peptid das Sequenzmotiv DB 4: RPRTRLRTHQNR (SEQ ID NO:1) oder aktive Fragmente davon.

In einer anderen nicht erfindungsgemäßen Ausführungsform umfasst das Peptid das Sequenzmotiv SHYRHISP (SEQ ID NO:3) oder aktive Fragmente davon.

In einer weiteren nicht erfindungsgemäßen Ausführungsform umfasst das Peptid das Sequenzmotiv GISWQQSHHLVA (SEQ ID NO:4) oder aktive Fragmente davon.

In einer weiteren nicht erfindungsgemäßen Ausführungsform der vorliegenden Erfindung umfasst das Peptid das Sequenzmotiv PRTRLHTH (SEQ ID NO:5) oder aktive Fragmente davon.

In einer weiteren nicht erfindungsgemäßen Variante wird das Peptid ausgewählt aus der Gruppe, bestehend aus Peptiden mit den D-Aminosäure-Sequenzen : a) QSHYRHISPAQV (SEQ ID NO:6); b) QSHYRHISPDQV (SEQ ID NO:7); c) QSHYRHISPAR (SEQ ID NO:8); d) KSHYRHISPAKV (SEQ ID NO:9); e) RPRTRLHTHRNR (SEQ ID NO:10); i) RPRTRLHTHRTE (SEQ ID NO:11); und g) KPRTRLHTHRNR (SEQ ID NO:12); ferner offenbart sind Sequenzen, die sich von den Sequenzen a) bis g) um bis zu drei Aminosäuren unterscheiden ; sowie Sequenzen, die die Sequenzen a) bis g) sowie Sequenzen, die sich von den Sequenzen a) bis g) um bis zu drei Aminosäuren unterscheiden, umfassen.

In einer weiteren nicht erfindungsgemäßen Variante wird das Peptid ausgewählt aus der Gruppe, bestehend aus Peptiden mit den D-Aminosäure-Sequenzen : D3D3: rprtrlhthrnrrprtrlhthrnr (SEQ ID NO:13), aD3nwnD3: rprtrlhthrnrnwnrprtrlhthrnr (SEQ ID NO:14), doppel-D3-freie-Ntermini: (rprtrlhthrnr)2-PEG3 (SEQ ID NO:15), doppel-D3-freie-Ctermini: PEG5-(rprtrlhthrnr)2 (SEQ ID NO:16), oder doppel-D3-freie-Ntermini: (rprtrlhthrnr)2- (SEQ ID NO:63), doppel-D3-freie-Ctermini: (rprtrlhthrnr)2 (SEQ ID NO:64), DB 3: rpitrlrthqnr (SEQ ID NO: 65), RD 1: pnhhrrrrrrtl (SEQ ID NO: 67), RD 3: rrptlrhthnrr (SEQ ID NO: 68), D3-delta-hth: rprtrlrnr (SEQ ID NO:69), NT-D3: rprtrl (SEQ ID NO: 70), DB 1: rpitrlhtnrnr (SEQ ID NO: 71), DB 2:rpittlqthqnr (SEQ ID NO: 72), DB 5: rpitrlqtheqr (SEQ ID NO. 74), D3-delta-hth D3-delta-hth: rprtrlrnrrprtrlrnr (SEQ ID NO:75), RD 2-RD 2: ptlhthnrrrrrptlhthnrrrrr (SEQ ID NO: 76), DO 3: sgwhynwqywwk (SEQ ID NO:77), rprtrsgwhynwqywwkrnr (SEQ ID NO:78) und ptlsgwhynwqywwkrrrrr (SEQ ID NO:79).

Ferner offenbart sind Sequenzen, die sich von den oben genannten Sequenzen um bis zu drei Aminosäuren unterscheiden; sowie Sequenzen, die jede einzelne oder jede beliebisge Kombinatio der oben genannten Sequenzen umfassen.

In einer weiteren nicht erfindungsgemäßen Variante weisen die D-Peptide Fragmente der oben genannten Sequenzen auf oder weisen homologe Sequenzen zu den oben genannten Sequenzen auf.

Ferner offenbart sind auch nicht erfindungsgemäße definierte, homogene und stabile Präparationen von Standards zur Quantifizierung von pathogenen Aggregaten oder Oligomeren aus körpereigenen Proteinen, insbesondere zur Behandlung von Blut, Blutprodukten und/oder Organen verwendbar. Einsetzbar sind hier Standards zur Quantifizierung von Oligomeren oder pathogenen Aggregaten, die eine Proteinaggregationserkrankung oder eine amyloide Degeneration oder Proteinvielfaltungserkrankung kennzeichnen. Hierbei wird ein Polymer aus Polypeptidsequenzen aufgebaut, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung oder eine amyloide Degeneration oder Proteinfehlfaltungserkrankung kennzeichnen, wobei die Polymere nicht aggregieren.

Als nicht erfindungsgemäßer Standard im Sinne der vorliegenden Offenbarung wird eine allgemein gültige und akzeptierte, feststehende Bezugsgröße bezeichnet, die zum Vergleichen und Bestimmen von Eigenschaften und/oder Menge dient, insbesondere zur Bestimmung der Größe und Menge von pathogenen Aggregaten aus körpereigenen Proteinen. Der Standard im Sinne der vorliegenden Erfindung kann zum Kalibrieren von Geräten und/oder Messungen verwendet werden.

Im Sinne der vorliegenden Offenbarung können unter dem Begriff "Proteinaggregationserkrankung" auch amyloide Degenerationen und Proteinfehlfaltungserkrankungen zusammengefasst werden. Beispiele solcher Krankheiten und die damit verbundenen körpereigenen Proteine sind: A-Beta- und Tau-Protein für AD, alpha-Synuclein für Parkinson, Amylin für Diabetes oder Prion-Protein für Prion-Erkrankungen, zum Beispiel wie die humane Creutzfeldt-Jakob-Krankheit (CJD), die Schafskrankheit Scrapie und die bovine spongiforme Enzephalopatie (BSE).

Unter dem Begriff "entsprechender Teilbereich" körpereigener Proteine ist jene Peptidsequenz zu verstehen, die gemäß den erfindungsgemäßen Definitionen eine identische oder mit der angegebenen Prozentzahl homologe Peptidsequenz eines Monomers, aus dem die erfindungsgemäßen Standards aufgebaut werden, aufweist.

Wesentlich für die Standards ist, dass die Standards nicht aggregieren, bevorzugt durch die Verwendung von monomeren Sequenzen, die nicht aggregieren, da der "entsprechender Teilbereich" körpereigener Proteine für die Aggregation nicht verantwortlich ist, oder die durch Blockierung der für die Aggregation verantwortlichen Gruppen nicht aggregieren.

Aggregate im Sinne der vorliegenden Offenbarung sind
- Partikel die aus mehreren, bevorzugt gleichen Bausteinen bestehen, die nicht kovalent miteinander verbunden sind und/oder
- nicht-kovalente Zusammenlagerungen mehrerer Monomere.

In einer nicht erfindungsgemäßen Ausführung besitzen die Standards eine genau definierte Anzahl von Epitopen, die kovalent miteinander verknüpft sind (unmittelbar oder über Aminosäuren, Spacer und/oder funktionelle Gruppen) für die Bindung der entsprechenden Sonden.

Sonden im Sinne der Offenbarung werden ausgewählt aus der Gruppe bestehend aus: Antikörper, Nanobody und Affibody.

Die Zahl der Epitope wird dadurch bestimmt, dass eine Polypeptid-Sequenz verwendet wird, die bezüglich ihrer Sequenz identisch mit jenem Teilbereich der körpereigenen Proteine ist, die ein Epitop bildet bzw. eine Homologie von mindestens 50 % mit diesem Teilbereich aufweist, und dabei die biologische Aktivität des Epitopes besitzt.

In einer weiteren nicht erfindungsgemäßen Ausführung handelt es sich bei den Epitopen um Epitope des A-Beta-Peptids ausgewählt aus den Teilbereichen A-Beta 1-11, A-Beta 3-11 oder pyroGluA-Beta 3-11, zum Beispiel des humanen N-terminale Epitops (mit folgender Sequenz: DAEFRHDSGYE (1-11) (SEQ ID NO:17)).

Das Standardmolekül ist ein Polymer aus den oben definierten Polypeptid-Sequenzen. Unter Oligomer ist ein Polymer aus 2, 3, 4, 5, 6, 7, 8, 9, 10, 11, 12, 13, 14, 15, 16, 17, 18, 19 oder 20 Monomeren (unter Monomer ist die o.g. Polypeptid-Sequenz zu verstehen) gebildet, oder Vielfache davon, bevorzugt 2 - 16, 4-16, 8-16, besonders bevorzugt 8 oder 16, oder Vielfache davon.

In einer nicht erfindungsgemäßen Alternative sind die Standards wasserlöslich.

In einer nicht erfindungsgemäßen Alternative sind die Standards aus gleichen PolypeptidSequenzen aufgebaut.

In einer weiteren nicht erfindungsgemäßen Alternative sind die Standards aus unterschiedlichen Polypeptid-Sequenzen aufgebaut.

In einer nicht erfindungsgemäßen Alternative werden solche oben definierte PolypeptidSequenzen in einer linearen-Konformation aneinandergereiht.

In einer nicht erfindungsgemäßen Alternative werden solche oben definierte PolypeptidSequenzen zu einem verzweigten, erfindungsgemäßen Oligomer aneinandergereiht.

In einer nicht erfindungsgemäßen Alternative werden solche oben definierte PolypeptidSequenzen zu einem cross-linked, Oligomer aneinandergereiht.

Verzweigte oder cross-linked, Oligomere könne durch Verknüpfung einzelner Bausteine mittels Lysin oder mittels Click-Chemie hergestellt werden.

Die Offenbarung betrifft in einer Alternative ein nicht erfindungsgemäßes Standardmolekül, enthaltend oder aufgebaut aus Kopien des aminoterminalen Teils des A-Beta-Peptids, ausgewählt aus den Teilbereichen A-Beta 1-11, A-Beta 3-11, oder pyroGluA-Beta 3-11, zum Beispiel des humanen N-terminale Epitops (mit folgender Sequenz: DAEFRHDSGYE (1-11) (SEQ ID NO:17)).

Die Vervielfältigung der Epitope durch funktionelle Gruppen kann vor oder nach der Synthese der einzelnen Bausteine durchgeführt werden. Charakteristisch für die Standards ist die kovalente Verknüpfung der Polypeptid-Sequenzen.

Die einzusetzenden Polypeptid-Sequenzen können identisch mit der Sequenz des A-Beta-Volllängen-Peptids sein oder zeigen eine Homologie von 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % mit der Sequenz des A-Beta-Volllängen-Peptid.

Alternativ werden auch zum Aufbau der Standard-Moleküle Polypeptid-Sequenzen eingesetzt, die identisch mit einem Teilbereich des A-Beta-Volllängen-Peptids sind, bzw. eine Homologie von 50, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % mit einem Teilbereich des A-Beta-Volllängen-Peptids zeigen. Wesentlich für die eingesetzten Sequenzen ist ihre Eigenschaft nicht (oder nur gemäß den Bedingungen kontrolliert) zu aggregieren und/oder ihre die Aktivität als Epitop.

In einer weiteren nicht erfindungsgemäßen Ausführung sind die Standards als Dendrimere aufgebaut. Die Dendrimere sind aus den oben beschriebenen zu verwendenden PolypeptidSequenzen aufgebaut und können ein zentrales Gerüstmolekül enthalten.

Die nicht erfindungsgemäßen Dendrimere enthalten in einer Variante PolypeptidSequenzen, die eine Sequenz besitzen, die identisch mit einem Teilbereich des A-Beta-Peptids ist, oder eine mindestens 50 %ige Homologie zu dem entsprechenden Teilbereich zeigt.

Unter dem Begriff mindestens 50 %ige Homologie ist auch eine höhere Homologie zu verstehen, ausgewählt aus der Gruppe bestehend aus 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % zu verstehen.

Standards, vorteilhaft mit höherer Löslichkeit im Wässrigen als pathogene Aggregate oder Oligomere aus körpereigenen Proteinen, sind in einer nicht erfindungsgemäßen Ausführung aus Polypeptid-Sequenzen gebildet, die identisch mit dem N-terminalen Bereich des A-Beta-Peptids sind oder mindestens 50 %ige Homologie dazu aufweisen. Unter dem N-terminalen Bereich von einem A-Beta-Polypeptid ist die Aminosäuresequenz A-Beta 1 - 8, A-Beta 1 - 11, A-Beta 1 - 16, A-Beta 3-11 oder pyroGluA-Beta 3-11zu verstehen.

Ein einsetzbares Standardmolekül kann Epitope für mindestens 2, 3, 4, 5, 6, 7, 8, 9, 10 oder mehr verschiedene Sonde enthalten.

Epitope charakteristisch für verschiedene Sonden können in die Standards dadurch eingebaut werden, dass Polypeptid-Sequenzen verwendet werden, die identisch mit unterschiedlichen Bereichen des A-Beta-Peptids sind, bzw. mindestens eine 50 %ige Homologie dazu aufweisen, aber die Aktivität des entsprechenden Epitops besitzen.

In einer nicht erfindungsgemäßen Ausführung werden hierzu Polypeptidsequenzen eingesetzt, die identisch oder eine 50 %ige Homologie mit dem N-terminalen Bereich des A-Beta-Polypeptids aufweisen, sowie Polypeptid-Sequenzen, die identisch bzw. mindestens eine 50 %ige Homologie mit dem C-Terminus des A-Beta-Polypeptids aufweisen.

In einer nicht erfindungsgemäßen Ausführung enthalten die Standardmoleküle sog. Spacer.

Unter einem Spacer ist ein Molekül zu verstehen, das über kovalente Bindungen in das Standardmolekül eingebaut ist, und bestimmte physikalische und/oder chemische Eigenschaften besitzt, durch welche die Eigenschaften des Standardmoleküls verändert werden. In einer Ausführung der Standards werden hydrophile oder hydrophobe, bevorzugt hydrophile Spacer, eingesetzt. Hydrophile Spacer werden ausgewählt aus der Gruppe der Moleküle, gebildet aus Polyethylenglycol, Zucker, Glycerin, Poly-L-Lysin oder beta-Alanin.

Die einsetzbaren Standards enthalten in einer nicht erfindungsgemäßen Alternative (weitere) funktionelle Gruppen.

Unter funktionellen Gruppen sind Moleküle zu verstehen, die kovalent an die Standardmoleküle gebunden sind. In einer Variante enthalten die funktionellen Gruppen Biotin-Gruppen. Dadurch wird eine starke kovalente Bindung an Streptavidin ermöglicht. Standardmoleküle enthaltend Biotin-Gruppen können so an Moleküle, enthaltend Streptavidin-Gruppen, gebunden werden. Falls die einsetzbaren Standardmoleküle Biotin und/oder Streptavidin-Gruppen enthalten, können so größere Standards zusammengebaut werden oder mehrere, ggf. unterschiedliche Standardmoleküle, an ein Gerüst gebunden werden.

In einer weiteren nicht erfindungsgemäßen Alternative enthalten die Standardmoleküle Farbstoffe zur spektralphotometrischen Bestimmung und/oder aromatische Aminosäuren. Aromatische Aminosäuren sind z.B. Tryptophane, Tyrosin, Phenylalanin oder Histidin, bzw. ausgewählt aus dieser Gruppe. Durch den Einbau von Tryptophan wird eine spektralphotometische Bestimmung der Konzentration von Standards in Lösung ermöglicht.

Weiterer Gegenstand sind ist der Einsatz von nicht erfindungsgemäßen Dendrimeren enthaltenden Polypeptiden, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung kennzeichnen.

Die Dendrimere können jede der oben beschriebenen Merkmale der Standards oder jede beliebige Kombination davon enthalten.

In einer nicht erfindungsgemäßen Alternative handelt es sich Dendrimere enthaltend eine genau definierte Anzahl von Epitopen für die kovalente Bindung von Sonden besitzt,

Dendrimer enthaltend Epitope des A-beta-Peptids.

Zur nicht erfindungsgemäßen Untersuchung von Blut, Blutprodukten und/oder Organen auf Amyloid-Beta-Oligomere kann ein Standard eingesetzt werden, zur Quantifizierung von pathogenen Aggregaten oder Oligomeren aus körpereigenen Proteinen, die eine Proteinaggregationserkrankung oder eine amyloide Degeneration oder Proteinfehlfaltungserkrankung kennzeichnen, dadurch gekennzeichnet, dass ein Polymer aus Polypeptidsequenzen aufgebaut wird, die bezüglich ihrer Sequenz identisch im entsprechenden Teilbereich mit den körpereigenen Proteinen sind oder eine Homologie von mindestens 50 % über den entsprechenden Teilbereich mit jenen körpereigenen Proteinen aufweisen, die eine Proteinaggregationserkrankung oder eine amyloide Degeneration oder Proteinfehlfaltungserkrankung kennzeichnen, wobei die Polymere nicht aggregieren.

Der nicht erfindungsgemäße Standard kann für die Bindung von Sonden eine genau definierte Anzahl von Epitopen besitzen, die kovalent miteinander verknüpft sind.

Der nicht erfindungsgemäße Standard kann auch Epitope des A-beta-Peptids enthalten und/oder die erfindungsgemäßen Sequenzen.

Zur Untersuchung von Blut, Blutprodukten und/oder Organen auf Amyloid-Beta-Oligomere kann auch folgendes nicht erfindungsgemäße Verfahren zur selektiven Quantifizierung von A-Beta-Aggregaten eingesetzt werden:
Das nicht erfindungsgemäße Verfahren umfassend die Immobilisierung von A-Beta-Fängermolekülen auf einem Substrat, Auftragen der zu untersuchenden Probe auf das Substrat, Hinzufügen von für die Detektion gekennzeichneten Sonden, die durch spezifische Bindung an ABeta-Aggregate diese markieren und Detektion der markierten Aggregate.

Dieses nicht erfindungsgemäße Verfahren zur selektiven Quantifizierung und/oder Charakterisierung von A-Beta-Aggregaten umfasst folgende Schritte:
a) Auftragen der zu untersuchenden Probe auf das Substrat,
b) Hinzufügen von für die Detektion gekennzeichneten Sonden, die durch spezifische Bindung an A-Beta-Aggregate diese markieren und
c) Detektion der markierten Aggregate, wobei
Schritt b) vor Schritt a) durchgeführt werden kann.

Es werden auch nicht erfindungsgemäße Standards zur Verfügung gestellt, die eine exakte und quantitative Bestimmung von pathogenen Aggregaten oder Oligomeren aus körpereigenen Proteinen möglich machen. Die nicht erfindungsgemäßen Standards sollen als interne oder externe Standards einsetzbar sein.

Ferner können genau definierte, homogene und stabile Präparationen von nicht erfindungsgemäßen Standards zur Quantifizierung von pathogenen Aggregaten oder Oligomeren aus körpereigenen Proteinen verwendet werden.

Nicht erfindungsgemäße Standards zur Quantifizierung von Oligomeren oder pathogenen Aggregaten, die eine Proteinaggregationserkrankung oder eine amyloide Degeneration oder Proteinfehlfaltungserkrankung kennzeichnen, sind dadurch gekennzeichnet, dass ein Polymer aus Polypeptidsequenzen aufgebaut wird, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung oder eine amyloide Degeneration oder Proteinfehlfaltungserkrankung kennzeichnen, wobei die Polymere nicht aggregieren.

Der nicht erfindungsgemäße Standard kann dadurch gekennzeichnet sein, dass dieser eine genau definierte Anzahl von Epitopen, die kovalent miteinander verknüpft sind, für die Bindung von Sonden besitzt.

Der nicht erfindungsgemäße Standard kann ferner dadurch gekennzeichnet sein, dass er Epitope des A-beta-Peptids und/oder die Sequenzen enthält.

Die nicht erfindungsgemäßen Standards werden als Fängermoleküle eingesetzt. In einer Alternative werden die Standards zur Entfernung und/oder Enttoxifizierung von Amyloid-Beta-Oligomere in Blut, Blutprodukten und/oder Organen verwendet.

Ferner offenbart ist ein nicht erfindungsgemäßes Kit, welches Standard und/oder Sequenzen umfasst. Die Verbindungen und/oder Komponenten des Kits können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotiterplatte, absorbiert sein. Ferner kann das Kit Anweisungen für den Gebrauch des Kits für eine Beliebige der Ausführungsformen enthalten.

Gegenstand der vorliegenden Erfindung ist auch wie vorstehend definiert, wobei ein Kit zur selektiven Quantifizierung von A-Beta-Aggregaten und/oder zur Behandlung (in vitro) von Blut, Blutprodukten und/oder Organen enthaltend ein oder mehrere der folgenden Komponenten verwendete wird:

. Ein solches Kit enthält ein oder mehrere der folgenden Komponenten:
- Substrat aus Glas das mit einem hydrophoben Stoff beschichtet ist;
- Standard;
- Fängermolekül;
- Sonde;
- Substrat mit Fängermolekül;
- Lösungen;
- Puffer.

Die Verbindungen und/oder Komponenten des Kits, das in der vorliegenden Erfindung verwendet wird, können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotiterplatte, absorbiert sein. Ferner kann das Kit Anweisungen für den Gebrauch des Kits für eine Beliebige der Ausführungsformen enthalten.

In einer weiteren Variante des Kits, das in der vorliegenden Erfindung verwendet wird, sind auf dem Substrat die oben beschriebenen Fängermoleküle immobilisiert. Zusätzlich kann das KIT, das in der vorliegenden Erfindung verwendet wird, Lösungen und/oder Puffer enthalten. Zum Schutz der Dextranoberfläche und/oder der darauf immobilisierten Fängermoleküle können diese mit einer Lösung oder einem Puffer überschichtet werden.

Das Kit, das in der vorliegenden Erfindung verwendet wird, kann auch mindestens einen Standard zur Quantifizierung von pathogenen Aggregaten oder Oligomeren aus körpereigenen Proteinen, die eine Proteinaggregationserkrankung enthalten.

Verbindungen die erfindungsgemäß in einem Verfahren zur Behandlung (in vitro, ex vivo) von Blut, Blutprodukten und/oder Organen außerhalb des menschlichen oder tierischen Körpers indem Amyloid-Beta-Oligomere entfernt und/oder enttoxifiziert werden, sind nachfolgend dargestellt:
RD 2: ptlhthnrrrrr (SEQ ID NO: 66)

Weitere Polymere, D-Peptide, Antikörper, und/oder Verbindungen die in einem nicht erfindungsgemäßen Verfahren zur Behandlung (in vitro) von Blut, Blutprodukten und/oder Organen zur Entfernung und/oder Entoxifizierung von Amyloid-Beta-Oligomeren einsetzbar sind, sind nachfolgend dargestellt:
DB 4: rprtrlrthqnr (SEQ ID NO:1)
D3D3: rprtrlhthrnrrprtrlhthrnr (SEQ ID NO:13)
D3nwnD3: rprtrlhthrnrnwnrprtrlhthrnr (SEQ ID NO:14)
doppel-D3-freie-Ntermini: (rprtrlhthrnr)₂-PEG3 (SEQ ID NO:15)
doppel-D3-freie-Ctermini: PEG5-(rprtrlhthrnr)₂ (SEQ ID NO:16)
kqhhveygsdhrfead (SEQ ID NO:2)
shyrhisp (SEQ ID NO:3)
giswqqshhlva (SEQ ID NO:4)
prtrlhth (SEQ ID NO:5)
qshyrhispaqv (SEQ ID NO:6)
qshyrhispdqv (SEQ ID NO:7)
qshyrhispar (SEQ ID NO:8)
kshyrhispakv (SEQ ID NO:9)
rprtrlhthrnr (SEQ ID NO:10)
rprtrlhthrte (SEQ ID NO:11)
kprtrlhthrnr (SEQ ID NO: 12)
daefrhdsgye (SEQ ID NO:17)
hhghspnvsqvr (SEQ ID NO: 18)
gsfstqvgslhr (SEQ ID NO: 19)
htgtqsyvprl (SEQ ID NO:20)
tlayaraymvap (SEQ ID NO:21)
tlayaraymvap (SEQ ID NO:22)
atpqndlktfph (SEQ ID NO:23)
tqpetdllrvqf (SEQ ID NO:24)
citwpptglty (SEQ ID NO:25)
tfletgpiyadg (SEQ ID NO:26)
Ivppthrhwpvt (SEQ ID NO:27)
appgnwrnylmp (SEQ ID NO:28)
dnysnyvpgtkp (SEQ ID NO:29)
svsvgmkpsprp (SEQ ID NO:30)
slpnpfsvssfg (SEQ ID NO:31)
yvhnpyhlpnpp (SEQ ID NO:32)
crrlhtyigpvt (SEQ ID NO:33)
gatmkkmddhtv (SEQ ID NO:34)
Igktqklsdahs (SEQ ID NO:35)
ddqarpymaygp (SEQ ID NO:36)
gdtwvnmvsmvh (SEQ ID NO:37)
gytwvnmvsmvh (SEQ ID NO:38)
wtntvarlatpy (SEQ ID NO:39)
qtqalyhsrqvh (SEQ ID NO:40)
nsqtqtlhlfph (SEQ ID NO:41)
hntsanilhssh (SEQ ID NO:42)
shinptsfwpap (SEQ ID NO:43)
tfsnplymwprp (SEQ ID NO:44)
gpspfnpqptpv (SEQ ID NO:45)
fsdhksptpppr (SEQ ID NO:46)
stsvyppppsaw (SEQ ID NO:47)
yglptqansmql (SEQ ID NO:48)
hnrtdntyirpt (SEQ ID NO:49)
Iqqplgnnrpns (SEQ ID NO:50)
kpedsaaypqnr (SEQ ID NO:51)
rpedsvitktqnt (SEQ ID NO:52)
raadsgctptkh (SEQ ID NO:53)
rprtrlhthrnt (SEQ ID NO:54)
rprtrlhthtnv (SEQ ID NO:55)
rprtrlhthtnr (SEQ ID NO:56)
rprtrlhthrkq (SEQ ID NO:57)
rprtrlhtlrnr (SEQ ID NO:58)
rrrsplhthrnr (SEQ ID NO:59)
Irsprqrripri (SEQ ID NO:60)
rkrqlrmttprp (SEQ ID NO:61)
shyrhispaqk (SEQ ID NO:62)
doppel-D3-freie-Ntermini: (rprtrlhthrnr)2- (SEQ ID NO:63)
doppel-D3-freie-Ctermini: (rprtrlhthrnr)2 (SEQ ID NO:64)
DB 3: rpitrlrthqnr (SEQ ID NO: 65),
RD 1: pnhhrrrrrrtl (SEQ ID NO: 67)
RD 3: rrptlrhthnrr (SEQ ID NO: 68)
D3-delta-hth: rprtrlrnr (SEQ ID NO:69)
NT-D3: rprtrl (SEQ ID NO: 70)
DB 1: rpitrlhtnrnr (SEQ ID NO: 71),
DB 2:rpittlqthqnr (SEQ ID NO: 72),
D3: rprtrlhthrnr (SEQ ID NO: 73 ()
DB 5: rpitrlqtheqr (SEQ ID NO. 74)
D3-delta-hth D3-delta-hth: rprtrlrnrrprtrlrnr (SEQ ID NO:75)
RD 2- RD 2: ptlhthnrrrrrptlhthnrrrrr (SEQ ID NO: 76)
DO 3: sgwhynwqywwk (SEQ ID NO:77)
rprtrsgwhynwqywwkrnr (SEQ ID NO:78)
ptlsgwhynwqywwkrrrrr (SEQ ID NO:79)

Antikörper, die in einem nicht erfindungsgemäßen Verfahren zur Behandlung (in vitro) von Blut, Blutprodukten und/oder Organen zur Entfernung und/oder Entoxifizierung von Amyloid-Beta-Oligomeren einsetzbar sind, sind nachfolgend definiert:
Antikörper, die
a) an eine retro-inverse Sequenz des Amyloid beta-Peptids oder Amyloid beta-Peptid-Teilfragmenten binden und/oder
b) an die Multimerisierungsdomäne des Amyloid beta-Peptids binden und auch an das Amyloid beta-Peptid und/oder
c) an eine der oben genannten Sequenzen ausgewählt aus der Gruppe:
   SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID N0:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79 oder homologe Sequenzen davon;
   binden.

Hybrid-Verbindungen, die in einem nicht erfindungsgemäßen Verfahren zur Behandlung (in vitro) von Blut, Blutprodukten und/oder Organen zur Entfernung und/oder Entoxifizierung von Amyloid-Beta-Oligomeren einsetzbar sind, sind nachfolgend definiert:
- Hybrid-Verbindung der Formel A-B wobei A ein Aminopyrazol oder ein Derivat davon ist und B ein Peptid und A und B unmittelbar oder mittels eines Linkers kovalent miteinander verbunden sind.
- Hybrid-Verbindung, dadurch gekennzeichnet, dass B ein D-Peptid ist, dadurch gekennzeichnet, dass das D-Peptid ausgewählt ist aus den oben genannten Sequenzen .
- Hybrid-Verbindung der Formel A-B, dadurch gekennzeichnet, dass

A ausgewählt ist aus der Gruppe der Verbindungen bestehend aus: 3-Aminopyrazol-5-carbonsäure, Derivate davon mit ersetzter heterocyclischer CH-Gruppe gegen - CR- oder -N-, -O-, -S-, 3-Aminopyrazol-5-carbonsäure-Dimer sowie -Trimer oder -Tetramer.

B ausgewählt ist aus der Gruppe der Verbindungen bestehend aus:
D3-Peptid, SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79;
der Linker ausgewählt ist aus der Gruppe der Verbindungen bestehend aus:
   kein Linker, GABA, TEG, TEG-Dimer, PEG, alpha-Aminosäuren, alpha-Amino-omegacarbonsäure.

Durch das erfindungsgemäße Verfahren werden pathogene Partikel, Amyloid-Beta-Oligomere entfent und/oder enttoxifiert, also in eine unschädliche Form überführt oder vernichtet. Somit wird eine weiter Infektion durch Verbleib und Vermehrung der pathogenen (amyloidogenen) Partikel vermieden. Dabei treten synergetische Effekte auf, ausgelöst durch die erfindungsgemäß einzusetzenden Verbindungen. So führen insbesondere die Ko-Aggregate aus Amyloid-Beta-Peptiden (-Oligomeren) und den erfindungsgemäß einzusetzenden Verbindungen als unschädliche Form zu einer späteren Verhinderung oder Reduzierung weiteren oder neuen Bildung von A-Beta-Aggeregaten.+

Das erfindungsgemäße Verfahren ist also sicherer als die aus dem Stand der Technik bekannten Verfahrten, da keine absolut restlose Entfernung der pathogegen, toxischen und/oder infektiösen Partikeln erfolgen muß. Es findet ein Enttoxifizierung durch Überführung in unschädliche Formen statt, die sogar eine spätere Infektion, also die Bildung von A-Beta-Aggeregaten, verhindern oder reduzieren kann.

### Beispiele (nicht erfindungsgemäß):

### 1. ThT-Seedingassay: Grundlagen

Amyloidogene Peptide besitzen die Fähigkeit, amyloide Fibrillen zu bilden. Dies kann mit einer gewissen Wahrscheinlichkeit spontan geschehen. Sind keine amyloiden "Keime" vorhanden, kann es einige Zeit dauern, bis die ersten Amyloid-Fibrillen gebildet werden, deren Bildung und Vermehrung mit Hilfe der Fluoreszenz von Thioflavin T (ThT) quantitativ verfolgt werden kann.

ThT interagiert mit A-Beta Fibrillen und der Fibrillen-Farbstoff-Komplex zeigt eine erhöhte Fluoreszenz (λem: 450 nm, λex: 490 nm). Die Zeit bis das ThT-Signal zu steigen beginnt, wird "lag phase" genannt. Diese "lag phase" kann vermieden oder stark verkürzt werden, wenn man amyloide "Keime", auch "seeds" genannt, zum Aggregationsansatz hinzu gibt. Ein bekanntes Beispiel ist das Hinzugeben von Prion-haltigem Hirnmaterial zu einer Lösung von monomerem rekombinanten Prionprotein, das daraufhin mit einer deutlich verkürzten "lag phase" ThT-positive Fibrillen bildet. Ein weiteres Beispiel besteht im Hinzufügen einer kleinen Menge von Abeta-Amyloid-Fibrillen zu einer Lösung von nicht-aggregiertem A-Beta-Peptid (Abeta). Auch dabei wird die "lag phase" zur Bildung ThT-positiver Abeta-Fibrillen deutlich verkürzt. Dadurch erlaubt es dieser Test (genannt "ThT-Seedingassay"), beliebige Substanzen oder Substanzgemische auf ihren Gehalt an Keim-fähigen Amyloiden zu untersuchen. Enthält das dem Aggregationsansatz hinzugegebene Substanzgemisch "seed"-fähige Amyloide, wirkt sich dies im Fehlen oder Verkürzen der "lag phase" aus. Diese in vitro-Eigenschaft wird oft analog zur Prion-ähnlichen Infektiosität oder Übertragbarkeit in vivo betrachtet.

Beim ersten Kontroll-Versuch wurde untersucht, ob vorgeformte Abeta-Aggregate als Aggregationskeime tatsächlich die "lag phase" der Aggregation von frisch gelöstem Abeta verkürzen. Dazu wurde die ThT-Fluoreszenz von frisch gelöstem Abeta in Abwesenheit und Anwesenheit von vorgeformten Abeta-Aggregaten verfolgt. Anschließend wurden Abeta-Aggregate präpariert, die aus einer Mischung aus Abeta(1-42) und einer Inhibitor-Substanz (in diesem Beispiel je eines der D-Peptide D3, DB1, DB2, DB3, DB4, DB5) gebildet wurden, die die Bildung von amyloiden Fibrillen stören soll. Die so gebildeten Abeta-Inhibitor-Koaggregate wurden genau wie die amyloiden Abeta-Aggregate zu einem ThT-Seedingassay gegeben, um das verbliebene amyloide Potential dieser Koaggregate zu bestimmen.

### 2. ThT-Seedingassay: Experimentelle Details

Es wurde 20 µM A-Beta-Peptid (1-42) zusammen mit einer der Inhibitor-Substabnzen (20 µM) für 7 Tage bei 37 °C in 10 mM NaPi pH 7.4 vorinkubiert. Anschließend wurde die Probe zentrifugiert (20 min, 16.100 x g), das Aggregat-Pelett 3x gewaschen und in 10 mM NaPi pH 7.4 resuspendiert. In Analoger weise wurden Aß-Fibrillen ohne Inhibitor als positiv-Kontrolle hergestellt. Direkt vor dem eigentlichen ThT-Seedingassay wurde frisches Aß (20 µM in 10 mM NaPi pH 7.4) mit den resuspendierten Aggregationskeimen, die in Anwesenheit oder Abwesenheit der Inhibitor-Substanzen entstanden waren, vermischt (80:20 Volumenanteile) und 10 µM ThT dazugegeben. Als Referenz diente frische Aß-Lösung, die mit Pufferlösung ohne Aggregationskeimen vermischt wurde (80:20 Volumenanteile) und 10 µM ThT enthielt. 50 µl der jeweiligen Reaktionslösung wurden in eine Vertiefung einer schwarzen 384-Well-Mikrotiterplatte pipettiert. Die ThT-Fluoreszenz wurde alle 30 min über 20 h bei einer Anregungswellenlänge von 440 nm und einer Emissionswellenlänge von 490 nm gemessen. Für die Auswertung wurde die Fluoresnzenzintensität durch Subtraktion der 20% zugesetzten Aggregationskeime korrigiert und der Mittelwert berechnet. Es wurde eine achtfache Bestimmung durchgeführt.

### 3. Durchführung:

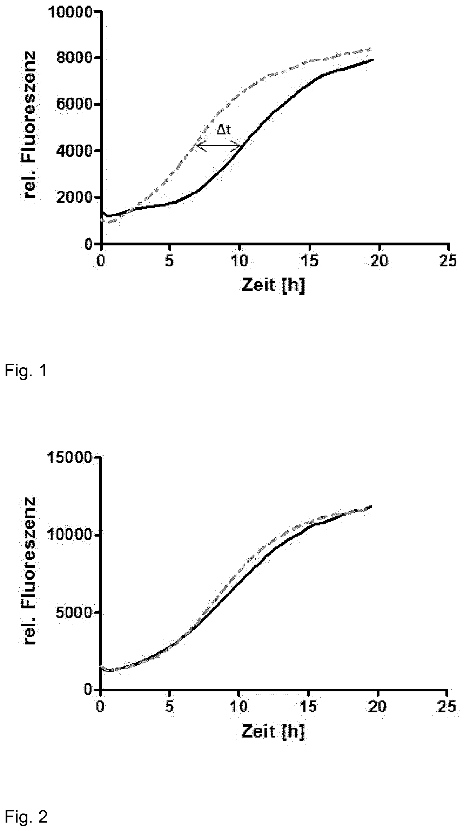

Fig. 1 zeigt den Zeitverlauf der ThT-Fluoreszenz in Abwesenheit (durchgezogene Linie) und Anwesenheit von vorgeformten Abeta-Aggregaten, die ohne Zusatz von Inhibitor-Substanzen entstanden sind.
   Es ist klar zu erkennen, dass diese Aggregate die Aggregation von frischem Abeta deutlich beschleunigen (Δt von etwa 4 h), also eindeutig einen Seeding-Effekt zeigen.
Fig. 2 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz D3 entstanden sind.
   Es ist klar zu erkennen, dass diese Aggregate die Aggregation von frischem Abeta nicht beschleunigen können, also eindeutig keinen Seeding-Effekt zeigen.

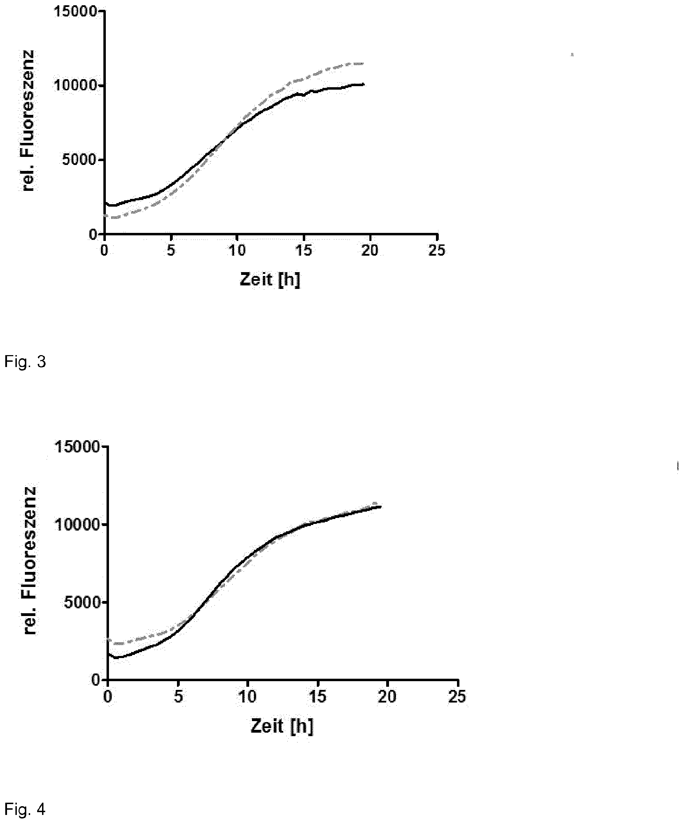

Fig. 3 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB1 entstanden sind.
   Es ist klar zu erkennen, dass diese Aggregate die Aggregation von frischem Abeta nicht beschleunigen können, also eindeutig keinen Seeding-Effekt zeigen.
Fig. 4 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB2 entstanden sind.
   Es ist klar zu erkennen, dass diese Aggregate die Aggregation von frischem Abeta nicht beschleunigen können, also eindeutig keinen Seeding-Effekt zeigen.

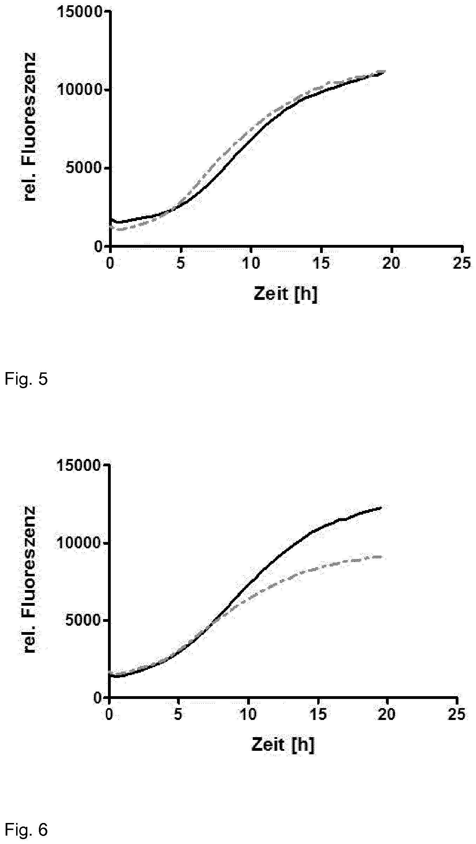

Fig. 5 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB3 entstanden sind.
   Es ist klar zu erkennen, dass diese Aggregate die Aggregation von frischem Abeta nicht beschleunigen können, also eindeutig keinen Seeding-Effekt zeigen.
Fig. 6 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB4 entstanden sind.
   Es ist klar zu erkennen, dass diese Aggregate die Aggregation von frischem Abeta nicht beschleunigen können, also eindeutig keinen Seeding-Effekt zeigen. Darüber hinaus scheinen die DB4-Abeta-Koaggregate sogar die Bildung von ThT-positiven Abeta-Aggregaten in der späteren Phase zu reduzieren.

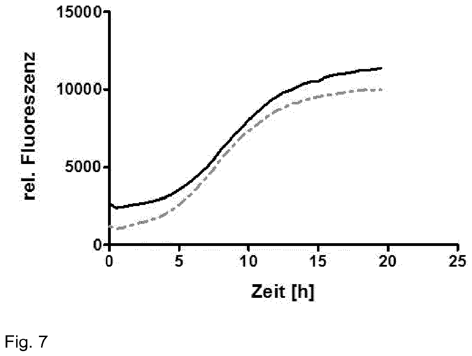

Fig. 7 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB5 entstanden sind.

Es ist zu erkennen, dass diese Aggregate die Aggregation von frischem Abeta nicht beschleunigen können, also eindeutig keinen Seeding-Effekt zeigen.

### 4. Zusammenfassung der Ergebnisse:

Alle getesteten D-Peptide bildeten mit Abeta Aggregate, die nicht mehr in der Lage waren, die "lag phase" der Aggregation von frisch gelöstem Abeta zu verkürzen. Diese Ko-Aggregate sind also nicht amyloidogen.

## Patentansprüche

1. Verfahren zur Behandlung (in vitro, ex vivo) von Blut, Blutprodukten und/oder Organen außerhalb des menschlichen oder tierischen Körpers indem Amyloid-Beta-Oligomere entfernt und/oder enttoxifiziert werden, **dadurch gekennzeichnet, dass** eine Verbindung enthaltend oder bestehend aus Peptid RD2, mit der Sequenz ptlhthnrrrrr gemäß SEQ ID NO: 66 eingesetzt wird.

2. Verfahren nach Anspruch 1 **dadurch gekennzeichnet, dass** ein Polymer eingesetzt wird enthaltend als eine Monomer-Einheit RD2 und als mindestens eine weitere Monomer-Einheit ein Peptid ausgewählt aus der Gruppe enthaltend:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ I0 NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ I0 NO:77, SEQ ID NO:78 und SEQ ID NO:79.

3. Verfahren nach einem der vorangehenden Ansprüche **dadurch gekennzeichnet, dass** die Verbindungen aus Anspruch 1 bis 2 als Fängermoleküle auf einem Träger angeordnet sind, über das eine das Blut, die Blutprodukte und/oder Organ enthaltende Probe geleitet wird.

4. Verfahren nach Anspruch 3 **dadurch gekennzeichnet, dass** die Fängermoleküle auf Beads fixiert sind.

5. Verfahren nach Anspruch 3 **dadurch gekennzeichnet, dass** die Fängermoleküle auf Nanomagneten immobilisiert sind.

6. Verfahren nach Anspruch 3 **dadurch gekennzeichnet, dass** die Fängermoleküle in einem Dialysesystem angeordnet sind.

7. Verfahren nach Anspruch 3 **dadurch gekennzeichnet, dass** der Träger für die Fängermoleküle aus einem biokompatiblen Material ist.

8. Verfahren nach Anspruch 3 **dadurch gekennzeichnet, dass** die Träger Membrane, Filter, Filterschwämme, Beads, Stäbe, Seile, Säule, Hohlfasern sind.

9. Verfahren nach einem der vorangehenden Ansprüche **dadurch gekennzeichnet, dass** ein Kit zur selektiven Quantifizierung von A-Beta-Aggregaten und/oder zur Behandlung (in vitro) von Blut, Blutprodukten und/oder Organen enthaltend ein oder mehrere der folgenden Komponenten:
- Substrat aus Glas das mit einem hydrophoben Stoff beschichtet ist;
- Standard;
- Fängermolekül;
- Sonde;
- Substrat mit Fängermolekül;
- Lösungen;
- Puffer;
verwendet wird.

10. Verfahren nach einem der vorangehenden Ansprüche **dadurch gekennzeichnet, dass** die Verbindungen aus Anspruch 1 bis 2 als Fängermoleküle ex vivo über und/oder durch ein Organ geleitet werden.

11. Verwendung einer Verbindung enthaltend oder bestehend aus Peptid RD2, mit der Sequenz ptlhthnrrrrr gemäß SEQ ID NO: 66 ex vivo als Fängermolekül für Amyloid-Beta-Oligomere.

12. Verwendung der Fängermoleküle gemäß Anspruch 11 bei der ex vivo Behandlung von Blut, Blutprodukten und/oder Organen zur Befreiung und/oder Inaktivierung von Aß-Oligomeren zur Prävention der Übertragung von Morbus Alzheimer.

13. Verwendnung eines Polymeres enthaltend als eine Monomer-Einheit RD2 und als mindestens eine weitere Monomer-Einheit ein Peptid ausgewählt aus der Gruppe enthaltend:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79. bei der ex vivo Behandlung von Morbus Alzheimer, Parkinson, *Creutzfeldt Jakob Disease* (CJD), Scrapie, bovine spongiforme Enzephalopatie (BSE), oder Diabetes.

## Claims

1. Process for the treatment (in vitro, ex vivo) of blood, blood products and/or organs outside of the human or animal body by removing and/or detoxifying amyloid beta oligomers, **characterized in that** a compound containing or consisting of peptide RD2 having the sequence ptlhthnrrrrr according to SEQ ID NO: 66 is used.

2. Process according to claim 1, **characterized in that** a polymer is used comprising as a monomer unit RD2 and as at least one further monomer unit a peptide selected from the group containing:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO: 17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO: 33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO: 49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 and SEQ ID NO:79.

3. Process according to one of the preceding claims, **characterized in that** the compounds of claim 1 to 2 are arranged as capture molecules on a carrier over which a sample containing the blood, the blood products and/or organ is passed.

4. Process according to claim 3, **characterized in that** the capture molecules are fixed on beads.

5. Process according to claim 3, **characterised in that** the capture molecules are immobilised on nanomagnets.

6. Process according to claim 3, **characterized in that** the capture molecules are arranged in a dialysis system.

7. Process according to claim 3, **characterized in that** the carrier for the capture molecules is made of a biocompatible material.

8. Process according to claim 3, **characterized in that** the carriers are membranes, filters, filter sponges, beads, rods, ropes, column, hollow fibres.

9. Process according to one of the preceding claims, **characterized in that** a kit for the selective quantification of A-beta aggregates and/or for the treatment (in vitro) of blood, blood products and/or organs containing one or more of the following components:
- substrate of glass coated with a hydrophobic substance;
- standard;
- capture molecule;
- probe;
- substrate with capture molecule;
- solutions;
- buffer;
is used.

10. Process according to one of the preceding claims, **characterized in that** the compounds of claim 1 to 2 are passed ex vivo as capture molecules over and/or through an organ.

11. Use of a compound comprising or consisting of peptide RD2, having the sequence ptlhthnrrrrr according to SEQ ID NO: 66 ex vivo as a capture molecule for amyloid beta oligomers.

12. Use of the capture molecules according to claim 11 in the ex vivo treatment of blood, blood products and/or organs for the removal and/or inactivation of Aβ oligomers for the prevention of the transmission of Alzheimer's disease.

13. Use of a polymer containing as a monomer unit RD2 and as at least one further monomer unit a peptide selected from the group containing:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO: 17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO: 33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO: 49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 and SEQ ID NO:79. in the ex vivo treatment of Alzheimer's disease, Parkinson's disease, Creutzfeldt Jakob disease (CJD), scrapie, bovine spongiform encephalopathy (BSE), or diabetes.

## Revendications

1. Procédé pour traiter (in vitro, ex vivo) le sang, des produits sanguins et/ou des organes en dehors du corps humain ou animal, en éliminant et/ou en détoxifiant des oligomères amyloïdes bêta,
**caractérisé en ce que** l'on utilise un composé contenant ou consistant en le peptide RD2, ayant la séquence ptlhthnrrrrr selon SEQ ID NO:66.

2. Procédé selon la revendication 1,
**caractérisé en ce que** l'on utilise un polymère contenant en tant qu'unité monomère du RD2 et en tant qu'au moins une autre unité monomère un peptide choisi dans le groupe comprenant :
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 et SEQ ID NO:79.

3. Procédé selon l'une des revendications précédentes,
**caractérisé en ce que** les composés des revendications 1 à 2 sont disposés en tant que molécules de capture sur un support sur lequel est acheminé un échantillon contenant le sang, les produits sanguins et/ou l'organe.

4. Procédé selon la revendication 3,
**caractérisé en ce que** les molécules de capture sont fixées sur des billes.

5. Procédé selon la revendication 3,
**caractérisé en ce que** les molécules de capture sont immobilisées sur des nano-aimants.

6. Procédé selon la revendication 3,
**caractérisé en ce que** les molécules de capture sont disposées dans un système de dialyse.

7. Procédé selon la revendication 3,
**caractérisé en ce que** le support pour les molécules de capture est en un matériau biocompatible.

8. Procédé selon la revendication 3,
**caractérisé en ce que** les supports sont des membranes, des filtres, des éponges filtrantes, des billes, des tiges, des cordes, des colonnes, des fibres creuses.

9. Procédé selon l'une des revendications précédentes,
**caractérisé en ce que** l'on utilise un kit pour la quantification sélective des agrégats A-bêta et/ou pour le traitement (in vitro) du sang, des produits sanguins et/ou des organes, contenant un ou plusieurs des composants suivants :
- un substrat en verre revêtu d'une substance hydrophobe ;
- un étalon ;
- une molécule de capture ;
- une sonde ;
- un substrat avec une molécule de capture ;
- des solutions ;
- des tampons.

10. Procédé selon l'une des revendications précédentes,
**caractérisé en ce que** les composés des revendications 1 à 2 sont acheminés en tant que molécules de capture ex vivo sur et/ou à travers un organe.

11. Utilisation d'un composé contenant ou consistant en le peptide RD2, ayant la séquence ptlhthnrrrrr selon la SEQ ID NO:66, ex vivo en tant que molécule de capture des oligomères amyloïdes bêta.

12. Utilisation des molécules de capture selon la revendication 11 dans le traitement ex vivo du sang, des produits sanguins et/ou des organes pour la libération et/ou l'inactivation des oligomères Aβ afin de prévenir la transmission de la maladie d'Alzheimer.

13. Utilisation d'un polymère contenant en tant qu'unité monomère du RD2 et comme au moins une autre unité monomère un peptide choisi dans le groupe comprenant :
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEO ID NO:64, SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 et SEQ ID NO:79
dans le traitement ex vivo de la maladie d'Alzheimer, de la maladie de Parkinson, de la maladie de Creutzfeldt Jakob (MCJ), de la tremblante du mouton, de l'encéphalopathie spongiforme bovine (ESB) ou du diabète.
